# EUROPÄISCHE PATENTSCHRIFT

(11) **EP 3 821 528 B1**
(45) Veröffentlichungstag und Bekanntmachung des Hinweises auf die Patenterteilung: **05.01.2022**
(21) Anmeldenummer: 20701538.9
(22) Anmeldetag: 10.01.2020
(51) Int. Cl.: H02P 25/032, H02P 8/02, H02P 8/10, A61M 37/00, H01F 7/16

(54) **PIGMENTIERGERÄT MIT EINEM LINEARANTRIEB**
PIGMENTATION DEVICE WITH A LINEAR DRIVE
APPAREIL DE PIGMENTATION AVEC UN ENTRAÎNEMENT LINÉAIRE

(30) Priorität: 13.01.2019 DE 102019000135
(43) Veröffentlichungstag der Anmeldung: 19.05.2021
(73) Patentinhaber: Long-Time-Liner Conture Make-up GmbH, 80331 München (DE)
(72) Erfinder: BLANK, Anton, 70173 Stuttgart (DE)
(74) Vertreter: Kilian Kilian & Partner
(86) Internationale Anmeldenummer: PCT/EP2020/050583
(87) Internationale Veröffentlichungsnummer: WO 2020/144352

(56) Entgegenhaltungen:
- EP-A2- 0 967 316
- DE-A1- 19 509 195
- DE-A1-102016 008 129
- KR-A- 20180 066 514

## Beschreibung

Die Erfindung betrifft ein Pigmentiergerät mit einem Linearantrieb und dessen Verwendung.

### Hintergrund und Stand der Technik

Pigmentiergeräte sind anwendungsspezifisch ausgebildete Tätowiergeräte für das Anbringen von dauerhaftem Make-up, auch Permanent Make-up (PMU) genannt. Diese sind als Handgeräte ausgeführt um feine Tätowierungen vorwiegend in Gesichtshaut einzustechen. Für das Anbringen von PMU werden spitze Stechmittel, vorzugsweise Nadeln, verwendet, um flüssige Farben vorwiegend in Linienform in die Gesichtshaut einzustechen, wo diese dann eine längere Zeit verbleiben sollen.

Beim PMU, wie auch beim Tätowieren, müssen die Farben durch die Epidermis hindurch (oberste Hautschicht) in die obere Schicht der lebenden Dermis eingebracht werden, um eine längere Haltbarkeit aufzuweisen, da die Epidermis stetig innerhalb von ungefähr 4 Wochen vom Organismus erneuert wird. Aus der Dicke der Epidermis von 0,05-0,2mm und einer Einbringtiefe der Farben in die lebende Dermis von 0,05mm bis 0,1mm resultiert die gesamte Einstechtiefe in die Haut, die demnach ungefähr 0,1mm-0,3mm beträgt.

Die flüssigen Farben werden durch ein hin- und hergehendes Stechmittel eingebracht, dessen Spitze sich in der jeweils eingefahrenen Stellung aus einem Farbdepot benetzt und welche die Farbe beim Ausfahren in die Haut einsticht. Die Einstechtiefe in die Haut muss vom Anwender bestimmt werden, der durch Beobachtung der Nadelspitze und der Haut erkennt, ob diese die lebende Dermis erreicht.

Für das Einstechen der Farben ist eine hin- und hergehende Nadelbewegung erforderlich, die streng axial ohne radiale Komponenten verlaufen muss. Um das Stechmittel anzutreiben, werden in Pigmentiergeräten hauptsächlich drehende Motoren eingesetzt, deren rotierende Bewegung mit einer Vielzahl verschiedener Getriebearten in die axiale Hin- und Herbewegung umgesetzt wird. Am Markt gibt es auch Linearmotoren auf Basis des elektromagnetischen Tauchankerprinzips, die auf ein Getriebe verzichten können.

Allen am Markt befindlichen Antrieben für Pigmentiergeräte ist gemeinsam, dass deren resultierende Hin- und Herbewegung eine periodische, nahezu harmonische sinusförmige Kurvenform aufweist, die den physikalischen Schwingungsgesetzen folgt und deren maximale Bewegungsgeschwindigkeit, die für das Pigmentieren zur Verfügung steht, direkt vom Antriebshub und dessen Wiederholfrequenz abhängt.

Der Hub der Antriebe des Stands der Technik liegt trotz der geringen erforderlichen Einstechtiefen bei ungefähr 2mm. Der Grund für den im Vergleich zu den geringen Einstechtiefen recht großen Hub liegt darin begründet, dass für jede Stechbewegung eine erneute Benetzung der Stechmittelspitze in einem Farbdepot erforderlich ist, welches nur in der eingefahrenen Stellung des Stechmittels erreicht werden kann und das Farbdepot sich nicht zu nahe an der Gerätespitze befinden darf, damit der Anwender noch mit ausreichend Abstand des Farbdepots zur Haut arbeiten kann.

Die Haut ist ein elastisches Gewebe. Trifft ein Stechmittel auf eine Haut, so dellt sich diese in Abhängigkeit von der Einstechgeschwindigkeit entsprechend ihres Durchdringungswiderstands und ihrer Elastizität zunächst ein, um ab einer, für jede Hauteigenschaft und Hautregion individuellen Energie von der Nadelspitze durchstochen zu werden. Der Antrieb muss geeignete Eigenschaften in Bezug auf die Bandbreite der individuell vorkommenden Hauteigenschaften bereitstellen, um die Farben in die vorgegebene Tiefe der federnden Haut einzustechen. Bedeutende Eigenschaften des Antriebs sind seine Eigenvibrationen die sich direkt auf die Nadel übertragen, seine reproduzierbare Einstechgeschwindigkeit und die Wiederholfrequenz der Stechbewegung. Geringe axiale Eigenvibrationen gestatten es dem Anwender, die Einstechtiefe von 0,1-0,3mm während der Pigmentierung von Hand anzugehen und diese aufrecht zu erhalten, geringe radiale Eigenvibrationen sorgen für Linien ohne seitliches Verwackeln. Eine hohe Einstechgeschwindigkeit ermöglicht geringe Eindellungen der Haut und damit eine manuell besser konstant zu haltende Stechtiefe, welche die Qualität der Pigmentierung verbessert. Eine geringe Wiederholfrequenz schont die Haut, indem in Bezug auf die Verfahrgeschwindigkeit nur so viele aneinandergereihte Stiche gesetzt werden, wie für optisch geschlossen wirkende Linien erforderlich sind.

Die bekannten Antriebe/Handgeräte haben eine Gesamtmasse von durchschnittlich 80g und arbeiten in einem Frequenzband von 50-200Hz, wobei in der Praxis meist eine Frequenz um die 100Hz gewählt wird. Bei 100Hz, einem Hub von 2mm und den unvermeidlichen bewegten Massen des Getriebes oder Tauchankers treten erhebliche Beschleunigungskräfte auf, die sich in unerwünschten Axial- und/oder Radialvibrationen des leichten Handgeräts auswirken. Messungen haben gezeigt, dass die Vibrationsamplituden bekannter PMU-Geräte im Mittel mehr als 0,5mm betragen. Bei Einstechtiefen von nur 0,1-0,3mm sind solche Vibrationsamplituden für exaktes Arbeiten in der Tiefe und/oder in Bezug auf eine seitliche Konstanz für die Pigmentierqualität und die Hautschonung abträglich. Zudem wirken die axialen Vibrationen entgegen der Einstechgeschwindigkeit und verkleinern diese erheblich.

Die bekannten Antriebe/Handgeräte, stellen in Bezug auf die Einstechgeschwindigkeit ungenügende Eigenschaften zur Verfügung. Die periodischen nahezu harmonischen sinusförmigen Weg-Zeitverläufe weisen bei einer Einstechtiefe von 0,1-0,3mm nur noch minimale Einstechgeschwindigkeiten auf, da das Stechmittel sich schon kurz vor dem Umkehrpunkt der sinusförmigen Schwingung befindet und weitgehend abgebremst ist. Die zu geringe Einstechgeschwindigkeit führt aufgrund des Eindringwiderstands zu großen Eindellungen der Haut bis diese durchstochen werden kann. Der Anwender muss deshalb das Stechmittel durch sogenanntes Vorhalten tiefer führen, als gestochen werden soll, so dass durch die in ihrer jeweiligen individuellen Eigenfrequenz flatternde Hautpartie partiell zu tief oder zu flach durchstochen wird, je nachdem in welcher vertikalen Ebene sich die nicht synchron zur Einstechfrequenz flatternde Haut beim nächsten Einstich gerade befindet. Ein gleichmäßiger Farbeintrag in der Tiefe, gleichmäßige Farbintensität im Verlauf der pigmentierten Linie, sowie hautschonendes und schmerzarmes Pigmentieren sind aufgrund der Asynchronität zwischen Hautfrequenz und Stechfrequenz nur unzureichend möglich.

Für kleine Hauteindellungen ist eine möglichst schnelle Einstechgeschwindigkeit erforderlich, die beim Stand der Technik nur durch Einstellen hoher Frequenzen möglich ist. Eine hohe Frequenz steht jedoch im Widerspruch zu den ansteigenden Vibrationen. Hohe Frequenzen stehen zusätzlich im Widerspruch zu der Notwendigkeit, die Stechfrequenz für hautschonendes Pigmentieren zu senken. Bereits bei 50Hz Stechfrequenz und den üblichen Verfahrgeschwindigkeiten werden Hautwunden in Furchenform erzeugt, weil sich die einzelnen Einstiche stark überlappen. Es bleibt dem Anwender also nur ein Kompromiss, der sich in der praktischen Anwendung einer Stechfrequenz von ungefähr 100Hz eingespielt hat, die jedoch zu Vibrationen der Handgeräte von mehr als 0,5mm und für irreversible Hautschäden verantwortlich ist.

Ein Pigmentiergerät gemäß Oberbegriff des Patentanspruches 1 ist bekannt aus dem Patentdokument KR 2018 0066514 A. Weiterer Stand der Technik ist bekannt aus DE 195 09 195 A1, EP 0 967 316 A2 und DE 10 2016 008129 A1.

### Überblick über die Erfindung

Aufgabe der Erfindung ist es, ein Pigmentiergerät zu schaffen, das gegenüber dem Stand der Technik geringere Vibrationen aufweist und bei Kleinen Stechfrequenzen gleichzeitig höhere Einstechgeschwindigkeiten bereitstellt.

Diese Aufgabe wird mit einem Pigmentiergerät gemäß Patentanspruch 1, bzw. dessen Verwendung in Anspruch 5, gelöst.

Bevorzugte Ausführungsformen werden in abhängigen Ansprüchen 2-4 definiert.

### Ausführungsbeispiel der Erfindung

Weitere Vorteile, Merkmale und Einzelheiten der Erfindung ergeben sich aus der nachfolgenden Beschreibung eines bevorzugten Ausführungsbeispiels des Linearantriebs für Pigmentiergeräte sowie anhand der Zeichnungen, hierbei zeigen:
Fig. 1 eine schematische Darstellung eines erfindungsgemäßen Pigmentiergeräts mit einem Linearantrieb in Ruhestellung mit stromloser Spule 2.
Fig. 2 eine vergrößerte schematische Darstellung des erfindungsgemäßen versetzten Luftspalts des Pigmentiergeräts mit einem Linearantrieb zum Zeitpunkt des Einschaltens des Stroms in der Spule 2.
Fig. 3 eine schematische Darstellung des Pigmentiergeräts mit einem Linearantrieb nach Fig. 1, zum Zeitpunkt des Umkehrpunkts der Ausfahrbewegung mit stromloser Spule 2.

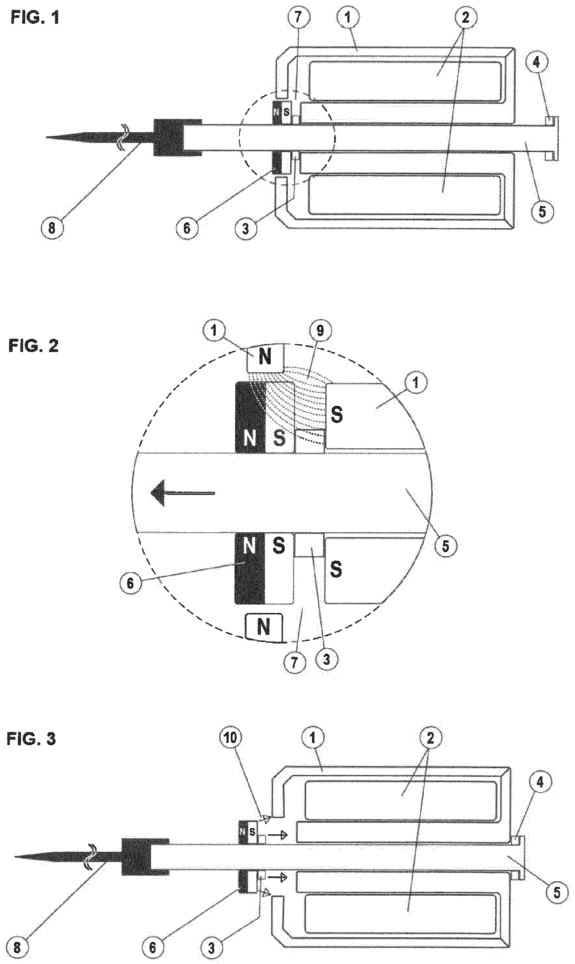

Fig. 1 zeigt schematisch ein Beispiel eines erfindungsgemäßen Pigmentiergeräts mit einem Linearantrieb in Ruheposition ohne elektrische Ansteuerung der Spule 2. Der Dauermagnet 6, der fest mit dem Anker 5 verbunden ist, liegt durch seine magnetischen Anziehungskräfte zum ferromagnetischen Stator 1 am Anschlag 3 an. Die Dicke des Anschlags 3 ist derart bemessen, dass der Dauermagnet 6 in der Ruheposition eine axial mittige Position zur Öffnung des Stators 1 einnimmt und dass die magnetische Anzugskraft des Dauermagneten 6 auf den Stator 1 durch Bestromen der Spule 2 erst kurz vor Erreichen des maximalen Betriebsstroms überwunden wird und daher eine Energiespeicherung im Dauermagneten 6 stattfindet, die für einen vorteilhaften Betrieb des Linearantriebs für Pigmentiergeräte genutzt wird. Der Anschlag 4 verhindert ein Herausfallen des Ankers 5 aus dem Stator 1 und begrenzt den Hub des Linearantriebs

Fig. 2 zeigt eine vergrößerte schematische Darstellung des erfindungsgemäßen versetzten Luftspalts zum Zeitpunkt des Einschaltens des Stroms in der Spule 2, welcher aufgrund der Induktivität der Spule 2 exponentiell ansteigt und ein ebenfalls exponentiell ansteigendes magnetisches Feld 9 im Luftspalt 7 hervorruft. Aufgrund der vorgesehenen und definiert ausgebildeten versetzten Anordnung des Luftspalts 7 durchflutet das elektromagnetische Feld 9 den Dauermagneten 6 in einer für die Erzeugung einer wirkungsgradstarken Abstoßungskraft günstigen Weise und erzeugt wegen der Konzentration des größten Teils der magnetischen Energie im Luftspalt 7 unter Berücksichtigung der in [19] beschriebenen Energiespeicherung im Dauermagneten 6 für ein nahezu schlagartiges Abstoßen des Dauermagneten 6 durch die elektromagnetische Kraft 9, so dass der Dauermagnet 6 zusammen mit dem Anker 5 ähnlich dem bekannten Knackfroscheffekt mit hoher Beschleunigung bis zum Anschlag 4 ausgefahren wird, was zu den vorteilhaften hohen Einstechgeschwindigkeiten führt. Die Summe der bewegten Massen von Anker 5, Magnet 6 und Stechmittel 8 liegt bei wenigen Gramm, so dass der Anker 5 bereits bei geringen Kräften eine hohe Beschleunigung erfährt und trotz des kleinen Hubs von beispielsweise 2mm hohe Geschwindigkeiten erreicht und sich die kleine Gesamtmasse vorteilhaft auf die Vibrationen des Gesamtsystems auswirkt. Die beim Ausfahren bremsende Kraftkomponente 10, die durch die Anziehungskraft des Dauermagneten 6 auf den ferromagnetischen Stator 1 ausgeübt wird, ist degressiv ausgelegt, so dass diese bremsende Kraft mit der Entfernung des Magneten 6 zum Stator 1 überproportional abnimmt, was eine wesentlich höhere Endgeschwindigkeit des Ankers 5 beim seinem Auftreffen auf den Anschlag 4 bewirkt, als bei den sinusförmigen Antrieben des Stands der Technik.

Die Variation der Geschwindigkeit einer Ausfahrbewegung wird über die Einschaltzeit der Spule gesteuert, die jedoch entgegen dem Stand der Technik stets zeitlich innerhalb der induktiven Anstiegsflanke des Spulenstroms beendet wird, mit dem Vorteil, den gesamten Einstellbereich bereits während der kurzen Anstiegsflanke steuern zu können. Auf diese Weise kann die Ausfahrgeschwindigkeit und die Stechfrequenz werksseitig vorteilhaft für das Pigmentieren vorgegeben und vom Anwender am Pigmentiergerät eingestellt werden.

Fig. 2 zeigt den Linearantrieb entsprechend Fig. 1 zum Zeitpunkt des Umkehrpunkts mit stromloser Spule 2, also zu dem Zeitpunkt, an dem der Anker gerade seine Bewegungsrichtung geändert hat, um in die Ruheposition zurückzukehren. Die eingerichtete degressive Kennlinie des Verlaufs der permanentmagnetischen Rückholkraft 10 hat sich beim Ausfahren vorteilhaft auf eine über dem Weg geringer werdende Minderung der Ausfahrgeschwindigkeit des Ankers 5 ausgewirkt. Beim Einfahren des Ankers 5 wirkt die vorher degressive bremsende Kraft 10 nun progressiv beschleunigend auf den Anker 5 und bewirkt eine von der vorangegangenen Ausfahrkennlinie unbeeinflusste Rücklaufzeit des Ankers 5 in die Ruheposition, so dass auch bei periodischer Ansteuerung eine vorteilhafte unharmonische Schwingung erzeugt wird. Für eine vorteilhaft schnelle Rückfahrbewegung des Ankers 5 in seine Ruheposition, kann die magnetische Kraft-Weg Kennlinie zwischen dem Dauermagneten 6 und dem Stator 2 mittels dessen geometrischer Formgebung und seiner ferromagnetischen Masse erfolgen.

## Patentansprüche

1. Pigmentiergerät mit einem Linearantrieb, welcher aufweist:
- einen Stator (1) und einen in diesem vorgesehenen und definiert versetzt gebildeten Luftspalt (7),
- eine elektrische Spule (2) innerhalb des Stators (1), die eingerichtet ist, mittels ihrer Bestromung eine Konzentration des magnetischen Flusses (9) im Luftspalt (7) zu bewirken,
- einen Anker (5), der ausgebildet ist, gleitende Axialbewegungen im Stator (1) auszuführen, und
- einen Dauermagneten (6), der fest mit dem Anker (5) verbunden ist,
**dadurch gekennzeichnet, dass**
der Linearantrieb ausgebildet ist, den Anker (5) als Reaktion auf eine Bestromung der Spule (2) und dem sich im Luftspalt (7) konzentrierenden elektromagnetischen Feld (9) hauptsächlich mittels magnetischer Abstoßung auf den Dauermagneten (6) aus einer Ruheposition, in der bei stromloser Spule (2) der Dauermagnet (6) den Anker (5) durch magnetische Anziehungskräfte zum Stator (1) hält, in eine ausgefahrene Position zu bewegen, in der der Anker (5) eine Umkehrung seiner Ausfahrbewegung vollzieht, wobei zu einem Zeitpunkt der Umkehrung der Ausfahrbewegung die Spule (2) stromlos ist und der Anker seine Ausfahrbewegung umkehrt, um aufgrund der Anziehungskräfte des Dauermagneten (6) in die Ruheposition zurückzukehren, und
eine Dicke eines Anschlags (3), an dem der Dauermagnet (6) und der Stator (1) in der Ruheposition anliegen, derart bemessen ist, dass in der Ruheposition die magnetischen Anziehungskräfte des Dauermagneten (6) auf den Stator (1) durch das Bestromen der Spule (2) erst kurz vor Erreichen einer maximalen elektromagnetischen Gegenkraft, also eines maximalen Betriebsstroms, überwunden werden, und eine im Dauermagneten (6) zu diesem Ablösezeitpunkt gespeicherte Energie schlagartig frei wird und den Anker (5) mit hoher Beschleunigung aus dem Stator (1) ausfährt.

2. Pigmentiergerät nach Anspruch 1, wobei eine Steuerung einer Ausfahrgeschwindigkeit des Ankers (5), mit der der Anker (5) aus dem Stator (1) ausfährt, mittels einer Einschaltdauer der Bestromung der Spule (2) bewirkt wird und die Einschaltdauer stets zeitlich während einer induktiven Anstiegsflanke des Spulenstroms beendet wird.

3. Pigmentiergerät nach Anspruch 1 oder 2, wobei eine unharmonische periodische Schwingung des Ankers (5) dadurch bewirkt wird, dass die Spule (2) stets vor Erreichen der ausgefahrenen Position des Ankers (5) stromlos geschaltet wird, so dass eine Einfahrbewegung des Ankers (5), mit der der Anker (5) in die Ruheposition zurückkehrt, weitgehend konstant ist und unabhängig von der über die Bestromung der Spule (2) steuerbaren Ausfahrbewegung des Ankers (5) wird.

4. Pigmentiergerät nach mindestens einem der vorangehenden Ansprüche, wobei eine Weg-Zeitkennlinie der Einfahrbewegung des Ankers (5) bei stromloser Spule (2), hervorgerufen durch die dauermagnetische Kraft-Wegkennlinie der magnetischen Anziehungskräfte (10) zwischen dem Dauermagneten (6) und dem Stator (2), durch Gestaltung einer Geometrie des Stators (1) und dessen magnetisch wirksame Masse derart ausgebildet ist, dass die Einfahrzeit des Ankers (5) minimalisiert ist.

5. Verwendung des Pigmentiergerätes nach mindestens einem der Ansprüche 1 bis 4 zum Anbringen von Permanent Make-up (PMU).

## Claims

1. A pigmentation device with a linear drive, comprising:
- a stator (1) and an air gap (7) which is provided in the stator (1) and is formed so as to be offset in a defined manner,
- an electric coil (2) within the stator (1), said coil (2) being configured to produce a concentration of the magnetic flux (9) in the air gap (7) as a result of the coil (2) being energized,
- an armature (5) which is designed to carry out sliding axial movements in the stator (1), and
- a permanent magnet (6), which is fixedly connected to the armature (5),
**characterized in that**
the linear drive is configured to move the armature (5), in response to the coil (2) being energized and the electromagnetic field (9) concentrated in the air gap (7), mainly by means of magnetic repulsion acting on the permanent magnet (6), from a rest position in which, when the coil (2) is deenergized, the permanent magnet (6) holds the armature (5) by magnetic attractive forces to the stator (1), into an extended position in which the armature (5) performs a reversal of its extension movement, wherein at a time of the reversal of the extension movement the coil (2) is deenergized and the armature reverses its extension movement in order to return to the rest position due to the attractive forces of the permanent magnet (6), and
a thickness of a stop (3), on which the permanent magnet (6) and the stator (1) lie in the rest position, is dimensioned in such a way that, in the rest position, the magnetic attractive forces of the permanent magnet (6) acting on the stator (1) are overcome by energizing the coil (2) only shortly prior to reaching the maximum electromagnetic counter-force, i.e. of the maximum operating current, and the energy which is then stored in the permanent magnet (6) at this release point is abruptly freed and extends the armature (5) out of the stator (1) with a high degree of acceleration.

2. The pigmentation device according to claim 1, wherein a control of an extension speed of the armature (5), with which the armature (5) extends from the stator (1), is effected by means of a switch-on duration of the coil being energized (2), and the switch-on duration is always terminated in time during an inductive rise of the coil current.

3. The pigmentation device according to claim 1 or 2, wherein a non-harmonic periodic oscillation of the armature (5) is effected by the coil (2) always being de-energized before the extended position of the armature (5) is reached, so that the retraction movement of the armature (5), with which the armature (5) returns to the rest position, is largely constant and becomes independent of the controlled extension movement of the armature (5) via the energization of the coil (2).

4. The pigmentation device according to at least any of the foregoing claims, wherein the non-energized path/time characteristic curve of the retraction movement of the armature (5), when the coil (2) is de-energized, caused by the permanent magnet force/path characteristic curve of the magnetic attractive forces (10) between the permanent magnet (6) and the stator (2), is formed by shaping the stator geometry (1) and the magnetically effective mass of said stator in such a way that the retraction time of the armature (5) is minimized.

5. Use of the pigmentation device according to at least any of claims 1 to 4 for application of Permanent Make-Up (PMU).

## Revendications

1. Appareil de pigmentation avec un entraînement linéaire, lequel présente :
- un stator (1) et un entrefer (7) prévu dans celui-ci et formé avec un décalage défini,
- une bobine électrique (2) à l'intérieur du stator (1), qui est mise au point pour entraîner au moyen de son alimentation en courant une concentration du flux magnétique (9) dans l'entrefer (7),
- un induit (5) qui est réalisé pour exécuter des mouvements axiaux par glissement dans le stator (1), et
- un aimant permanent (6), qui est relié de manière solidaire à l'induit (5),
**caractérisé en ce que**
l'entraînement linéaire est réalisé pour déplacer l'induit (5) en réaction à une alimentation en courant de la bobine (2) et au champ électromagnétique (9) se concentrant dans l'entrefer (7) principalement au moyen d'une répulsion magnétique sur l'aimant permanent (6) depuis une position de repos, dans laquelle en cas de bobine (2) sans courant, l'aimant permanent (6) maintient l'induit (5) par des forces d'attraction magnétiques par rapport au stator (1), dans une position sortie, dans laquelle l'induit (5) accomplit une inversion de son déplacement de sortie, dans lequel à un moment de l'inversion du déplacement de sortie, la bobine (2) est sans courant et l'induit inverse son déplacement de sortie pour retourner dans la position de repos en raison des forces d'attraction de l'aimant permanent (6), et
une épaisseur d'une butée (3), sur laquelle l'aimant permanent (6) et le stator (1) reposent dans la position de repos, est dimensionnée de telle manière que dans la position de repos, les forces d'attraction magnétiques de l'aimant permanent (6) sur le stator (1) sont surmontées par l'alimentation en courant de la bobine (2) seulement brièvement avant d'atteindre une contre-force électromagnétique maximale, donc un courant de fonctionnement maximal, et une énergie stockée dans l'aimant permanent (6) à un moment de déclenchement est brutalement libérée et fait sortir l'induit (5) hors du stator (1) avec une accélération élevée.

2. Appareil de pigmentation selon la revendication 1, dans lequel une commande d'une vitesse de sortie de l'induit (5), avec laquelle l'induit (5) sort du stator (1), est provoquée au moyen d'une durée d'activation de l'alimentation en courant de la bobine (2) et la durée d'activation se termine systématiquement dans le temps pendant un flanc ascendant inductif du courant de bobine.

3. Appareil de pigmentation selon la revendication 1 ou 2, dans lequel une oscillation périodique non harmonique de l'induit (5) est provoquée en ce que la bobine (2) est systématiquement commutée sans courant avant que l'induit (5) n'atteigne la position sortie si bien qu'un déplacement de rentrée de l'induit (5), avec lequel l'induit (5) retourne dans la position de repos, est largement constant et devient indépendant du déplacement de sortie de l'induit (5), pouvant être commandé par l'intermédiaire de l'alimentation en courant de la bobine (2).

4. Appareil de pigmentation selon au moins l'une quelconque des revendications précédentes, dans lequel une ligne caractéristique de temps de parcours du déplacement de rentrée de l'induit (5), en présence d'une bobine sans courant (2), provoquée par la courbe caractéristique de parcours de force magnétique permanente des forces d'attraction magnétiques (10) entre l'aimant permanent (6) et le stator (2), par configuration d'une géométrie du stator (1) et de sa masse active magnétiquement est réalisée de telle manière que le temps de rentrée de l'induit (5) est minimisé.

5. Utilisation de l'appareil de pigmentation selon au moins l'une quelconque des revendications 1 à 4 pour appliquer du maquillage permanent (PMU).
